Europäisches Patentamt

European Patent Office

Office européen des brevets

(11) Publication number: **0 442 522 A1**

# EUROPEAN PATENT APPLICATION

(21) Application number: **91102177.2**

(22) Date of filing: **15.02.91**

(51) Int. Cl.5: **C12N 1/12**, //(C12N1/12, C12R1:89)

(30) Priority: **16.02.90 JP 35511/90**

(43) Date of publication of application:
**21.08.91 Bulletin 91/34**

(84) Designated Contracting States:
**CH DE ES FR GB LI NL**

(71) Applicant: **BIOX CORPORATION**
**24-7, Higashinihonbashi 2-chome**
**Chuo-ku, Tokyo(JP)**

(72) Inventor: **Mutai, Masahiko**
**c/o Biox Corporation, 24-7,**
**Higashinihonbashi**
**2-chome, Chuo-ku, Tokyo(JP)**

(74) Representative: **Boeters, Hans Dietrich, Dr. et al**
**Boeters & Bauer Patentanwälte**
**Bereiteranger 15**
**W-8000 München 90(DE)**

(54) **Method for culturing a chlorella.**

(57) There is disclosed a method for culturing a chlorella aerobically in a culture medium containing an organic carbon source, which comprises using whey treated with a lactose-decomposing enzyme as the said organic carbon source. The disclosure described provides a method for culturing a chlorella wherein a chlorella of many various kinds can be cultured inexpensively with a high yield and a high multiplication rate from whey that is presently discarded as waste liquor.

**FIG.1**

V P (ml/l) vs Cultured Time (hr.)

o This Invention
x Comparative Example

Rank Xerox (UK) Business Services

FIELD OF THE INVENTION

The present invention relates to a method for culturing a chlorella aerobically by using enzymatically treated whey as an organic carbon source.

BACKGROUND OF THE INVENTION

Since chlorella cells contain 50% or more proteins, chlorella has potential as a possible food source and many related studies have been done.

To culture a chlorella, there are (1) a method wherein a chlorella is cultured by photosynthesis (autotrophism), (2) a method wherein a chlorella is cultured by causing the chlorella to utilize organic carbon aerobically in darkness (heterotrophism), and a method wherein the methods (1) and (2) are combined so that photosynthesis can be carried out in light and organic carbon can be utilized aerobically (mixotrophism). However, when photosynthesis is used, since there is a problem that the culturing is influenced by the weather, the method (2) is used industrially.

However, since, in any of the above culturing methods, the obtained chlorella is too expensive to be used as a food protein source, at present it is only used in health foods, as a food additive (the cells or the extract liquid is used as a colorant or a sapid component), and in feeds for culture and artificial feeds for silkworms and an inexpensive method for culturing a chlorella has been in need of development.

On the other hand, whey, also called milk serum, is a transparent light-yellow aqueous solution that is obtained by adding, to skim milk produced from milk by removing the fat, an acid or rennet so that casein, the main protein of milk, may be removed. The aqueous solution obtained by causing an acid or rennet to work directly on milk to remove curd made up of fat and casein is also called whey. Whey comprises mainly lactose, and it contains milk serum proteins, inorganic materials, and vitamins.

Whey is produced in a large amount as a by-product in the process of making, for example, cheese and casein from milk. Although part of the whey is recovered as whey powder and used as food and feed, most of it is discarded as waste liquor. In recent years, the production of cheese and casein has increased throughout the world, and as a result the amount of whey has increased. However, when whey is discarded as waste liquor, expensive waste liquor treatment facilities are required from the standpoint of the environmental protection standards, and therefore the development of an effective use of whey is desired.

Taking the above into consideration, recently a method for culturing a chlorella inexpensively by using whey has been suggested. This method is one wherein a chlorella capable of utilizing lactose is cultured under aerobic conditions in a whey culture medium to which nitrogen compounds and inorganic salts have been added.

However, the kinds of chlorellas capable of utilizing lactose in whey are only particular ones, and therefore in the above method a general chlorella could not be used without considering the kind. Further, the culturing method wherein chlorellas capable of utilizing lactose are used is slow in the multiplication rate of the chlorella, so that it has been difficult to culture a chlorella in a large amount industrially.

SUMMARY OF THE INVENTION

The present invention has been made taking the above problems into account and the object of the present invention is to provide a method for culturing a chlorella so that a chlorella can be industrially cultured by using whey to provide the chlorella inexpensively and also whey can be used effectively.

Other objects, features and advantages of the present invention will be apparent from the following detailed description taken in conjunction with the accompanying drawings.

BRIEF DESCRIPTION OF THE DRAWINGS

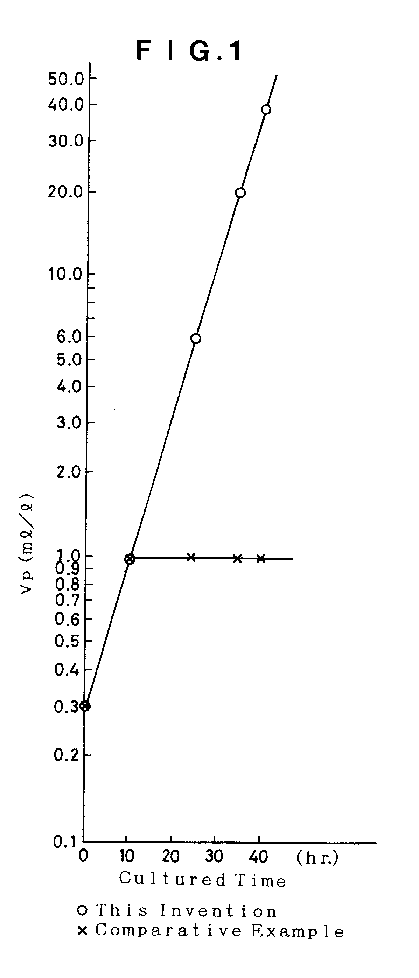

Fig. 1 is a diagram showing courses of culturing chlorella.

DETAILED DESCRIPTION OF THE INVENTION

To attain the above object, the present method for culturing a chlorella aerobically in a culture medium containing an organic carbon source is characterized in that, as the said organic carbon source, whey treated with a lactose-decomposing enzyme (lactase) is used.

The present invention will now be described in detail with reference to preferable modes.

As stated above, the present invention uses, as an organic carbon source, whey that has been treated with a lactose-decomposing enzyme to decompose the lactose in the whey into lactose and galactose.

As described above, whey, also called milk serum, is a transparent light-yellow aqueous solution that is obtained by adding, to skim milk produced from milk by removing the fat, an acid or rennet so that casein, the main protein of milk, may be removed. The aqueous solution obtained by causing an acid or rennet to work directly on milk to remove curd made up of fat and casein is also called whey. As kinds of wheys, there are, for example, cheese whey, casein whey, and lactose-fermented whey. Waste liquor obtained, for example, by heat treatment or ultrafiltration of whey to recover milk serum proteins is also called whey.

Whey contains 5 to 7% solids and the component composition, for example, of cheese whey, whose production is largest, is such that the water content is about 93.7%, the lactose content is about 4.50%, the nitrogen compound content is about 0.70%, the inorganic component content is about 0.60%, the fat content is about 0.30%, and the lactic acid content is about 0.20%, and water-soluble vitamins are also contained. The inorganic components are salts of potassium, sodium, calcium, phosphorus, magnesium, and others.

In the present invention, although whey produced as a by-product in the process of making, for example, cheese and casein can be used as it is, for example, in the case of whey whose lactose content is low, it may be used after increasing the lactose content by condensing it by ultrafiltration, reverse osmometry, or the like; one also can be used that is obtained by dissolving whey powder in water until a desired concentration is obtained, and whey can also be used wherein a condensed liquid of whey, powder whey, or lactose is added to increase the lactose concentration.

On the other hand, as lactose-decomposing enzymes, for example, $\beta$-galactosidases and galactosyltransferases can be mentioned. As these enzymes, enzymes that originate from, for example, Aspergillus oryzae, Aspergillus nigar, Kluyveromyces lactis, Kluyveromyces fragilis, and Bacillus circulans are known. Specifically, for example, "Lactase Y-AO" (that is a trade name and it is manufactured by Yakult Honsha Co., Ltd.), "Lactozym" (that is a trade name and it is manufactured by Novo Industrias), "Maxilact" (that is a trade name and it is manufactured by Gist-Brocades n.v.), and "Biolacta" (that is a trade name and it is manufactured by Daiwa Kasei Co., Ltd.) are on the market and can be readily obtained.

Whey may be pretreated with a lactose-decomposing enzyme to be used as a culture medium component before a chlorella is cultured, or a lactose-decomposing enzyme is may be added to a culture medium containing whey and the culturing of the chlorella and the treatment of the whey with the lactose-decomposing enzyme may be allowed to proceed simultaneously.

If whey is pretreated with a lactose-decomposing enzyme to be used as a culture medium component, the lactose decomposing enzyme is added to the whey and preferably they are reacted at 5 to 60°C for 2 to 24 hours. Although the amount of the enzyme to be added is not particularly limited, preferably the amount is 1 to 100 units per gram of the content of the lactose in the whey. However, in this case, since an equilibrium reaction takes place, it is difficult to decompose the lactose completely, so that the decomposition rate of the lactose in the whey is in the order of 70 to 80%.

In the case wherein a lactose-decomposing enzyme is added to a culture medium containing whey and the culturing of a chlorella and the treatment of the whey with an enzyme are caused to proceed simultaneously, since the enzyme may be allowed to work on the whey little by little by taking a lot of time, the amount of the enzyme may be about 1/10 to 1/100 of that used when whey is pretreated with an enzyme. Since the lactose in whey is decomposed with the enzyme and is consumed immediately as the organic carbon source of the chlorella, an equilibrium reaction does not take place, and almost all of the lactose in the whey is decomposed into glucose and galactose. According to the inventor's finding, when the treatment of whey by a lactose-decomposing enzyme and the culture of a chlorella are caused to proceed simultaneously, the multiplication of the chlorella is not inhibited by the presence of enzyme.

Thus, to cause the treatment of whey with an enzyme and the culturing of a chlorella to proceed simultaneously is more advantageous than to pretreat whey before the culturing of a chlorella, since in the former case the needed amount of an enzyme is small, the decomposition rate of lactose is high, and labor for pretreatment is not required.

It is possible to add, in addition to the above whey, for example, as auxiliary nutrients, inorganic salts of potassium, phosphorus, magnesium, iron, etc., a nitrogen source, such as urea and potassium nitrate, and a vitamin source, such as yeast extract, into the culture medium. These auxiliary nutrients are not necessarily required because whey contains inorganic salts of potassium, sodium, calcium, phosphorus, magnesium, etc. and water-soluble vitamins such as vitamins $B_1$, $B_2$, and C and nicotinic acid. However, to culture a chlorella, in particular an iron salt is required, so that it is preferable to add an iron salt, such as ferrous sulfate. Further, in some cases, depending on the kind of whey, it is preferable to add trace amounts of magnesium sulfate and metal salts of zinc, manganese, copper, boron, molybdenum, etc. Although nitrogen compounds are present in whey, since the amount thereof is short of the required amount of nitrogen compounds for the growth of a chlorella, it is preferable to cover this deficiency by adding, for example, urea, a nitrate, and an ammonium salt. With respect to the amount of nitrogen compounds to be added, if urea is used as nitrogen, the amount is preferably about 5% of lactose in the whey. Further when a nutrient rich in vitamins, such as yeast extract, is added, the multiplication rate of the chlorella and the yield thereof to lactose can be improved.

In the present invention, since lactose in whey is decomposed with a lactose-decomposing enzyme into glucose and galactose, which are used as an organic carbon source, the chlorella to be used is not a special one capable of utilizing lactose; rather it may be a common one that can multiply by using glucose and galactose. As the chlorella, many kinds of chlorellas, such as Chlorella vulgaris, Chlorella protothecoides, Chlorella regularis, and Chlorella pyrenoidosa can be mentioned, which can be taken out from outdoors and can be multiplied or can be obtained from the National Pollution Institute (Kokuritsu Kogai Kenkyusho).

To culture a chlorella, the culture medium prepared in the above manner is sterilized by heating it, preferably to about 120°C, for example, by treating it in an autoclave or by blowing live steam into it; then after cooling it, it is inoculated with a chlorella, and the culturing is effected aerobically in darkness. However, if the culturing and the treatment with a lactose decomposing enzyme are simultaneously effected, the enzyme is added to the culture medium after sterilization, for example, by filtering through a germ-removing filter. As a culturing vessel, a culturing tank wherein aerated culturing can be done may be used. Although irradiation with light is not particularly necessary, irradiation with light may be used to carry out photosynthesis. Preferably, the culturing conditions are kept such that the pH is 6 to 8, preferably 6 to 7, the temperature is 25 to 37°C, and the amount of air to be passed is 0.5 to 1 VVM (Volume Volume Minute), and when the pH lowers along with the process of the culturing, it is preferable to adjust the pH to 6 to 8 by adding NaOH (caustic soda) or the like. Although the time required for the culturing varies depending on the multiplication rate of the chlorella, the culturing may be stopped when the nutrients in the culture medium have been consumed sufficiently and the multiplication of the chlorella reaches a maximum.

After the completion of the culturing, the broth is subjected to centrifugation to separate the chlorella, followed by condensation, washing with water, and drying to obtain dried chlorella. The liquid after the culturing can also be used as it is, without separating the chlorella, as feed for cattle or fish.

In the present invention, since whey is treated with a lactose-decomposing enzyme to decompose lactose in the whey into glucose and galactose, which are used as an organic carbon source, the kind of the chlorella to be used is not a particular one capable of utilizing lactose, and any one capable of multiplying heterotrophically by using glucose and galactose can be used, so that many various common kinds of chlorellas can be used.

Since the kind of chlorella is not restricted, chlorellas high in capability of multiplication can be used, and since lactose in whey can be utilized well by treatment with an enzyme, the multiplication and yield of the chlorella can be made higher than the case wherein whey is used as it is to culture a chlorella capable of utilizing lactose.

Herein, if the culturing of the chlorella and the enzyme reaction are caused to proceed simultaneously by adding a lactose decomposing enzyme to the culture medium containing whey, the decomposition ratio of the lactose in whey can be increased further, the yield of the chlorella can be increased further, and the amount of required enzyme is less.

Additionally, since whey that is presently discarded as waste liquor at present is utilized, a chlorella can be cultured inexpensively and since whey is effectively utilized without being discarded as waste liquor, this culturing method can contribute to the protection of the environment.

Next, the present invention will be described in detail in accordance with examples, but it should be understood that these examples are not intended to limit the scope of the invention.

Example 1

Whey obtained in the process of making cheese was used. The composition of the whey was as follows:

Solids content
6.7 %
Water content
93.3 %
Lactose content
5.2 %
Nitrogen compound content
0.7 %
Inorganic component content
0.5 %
Fat content
0.1 %
Lactic acid content
0.2 %

A culture medium was prepared by adding the components shown below to the above-mentioned whey.

The components shown below were added to whey obtained in the process of making cheese to prepare a culture medium.

$KH_2PO_4$
0.5 g/ℓ
$MgSO_4 \cdot 7H_2O$
0.5 g/ℓ

FeSO₄·7H₂O

5 mg/ℓ

As solution

1 mℓ/ℓ

Yeast extract

2 g/ℓ

Urea

3.0 g/ℓ

The As solution was a solution prepared by mixing the following components:

H₃BO₃

2.86 g

MnCℓ₂·4H₂O

1.81 g

ZnSO₄·7H₂O

0.22 g

CuSO₄·5H₂O

0.08 g

Na₂MoO₄

0.021 g

Distilled water

1 ℓ

Concentrated sulfuric acid

1 drop

1 ℓ of the culture medium thus prepared was placed in a culturing tank, then after passing live steam thereinto to sterilize it at a high temperature of 120° C under high pressure for 20 min, "Lactase Y-AO" (which is a trade name and it is manufactured by Yakult Honsha Co., Ltd.), which is $\beta$-galactosidase, in an amount equivalent to 40 units dissolved in 5 mℓ of water and sterilized by filtering through a Millipore filter (pore size: 0.45 $\mu$m) was added thereto; as chlorella, Chlorella regularis was taken thereinto in an amount of Vp = 0.3 mℓ/ℓ, and the culturing of the chlorella and the decomposition of the whey with the enzyme were carried out in darkness under such conditions that the amount of air passed was 1 VVM (Volume Volume Minute), the temperature was 30° C, and the pH was 6 to 7. When the pH dropped during the culturing, NaOH (caustic soda) was added to keep the pH at 6 to 7.

The course of the culturing is shown as the line of -o-o- in Fig. 1.

Comparative Example 1

For comparison, Example 1 was repeated, except that the culture medium prepared as in Example 1 was used without adding lactose, thereby culturing the chlorella.

The course of the culturing is shown as the line of -x-x- in Fig. 1.

Fig. 1 is a diagram showing the course of the culturing wherein a chlorella is cultured according to the present method and the course of the culturing wherein a chlorella is cultured according to the method of Comparative Example 1, o-o showing the course of the culturing wherein a chlorella is cultured according to the present method and x-x showing the course of the culturing wherein a chlorella is cultured according to the method of Comparative Example 1.

In Fig. 1, the numbers on the abscissa indicate cultured time in hour of chlorella, and by Vp on the ordinate is meant packed cell volume (wet cell concentration) in 1 liter which, is abbreviated to PCV and indicates cell concentration. For example, Vp = 3 indicates that 1 liter of a suspension contains 3 mℓ of live cells (with respect to the method of the measurement, see "Sourui Jikken-ho," page 186, Nanko-do).

From the results shown in Fig. 1, it can be understood that when whey that has not been treated with an enzyme is used as an organic carbon source, the multiplication of the chlorella stops soon, whereas when a chlorella is cultured while whey is treated with an enzyme, even a chlorella not capable of utilizing lactose multiplies well by using the whey effectively.

Example 2

The pH of 1 ℓ of whey was adjusted to 6.5, 1,000 units of "Lactozym" (which is a trade name and is manufactured by Novo Industrias, which is $\beta$-galactosidases, was added thereto, and the whey was treated therewith at 40° C for 5 hours, thereby obtaining enzymatically treated whey.

The same components as in Example 1 were added to the enzymatically treated whey to prepare a culture medium, the culture medium was placed in a culturing tank, live steam was passed thereinto to sterilize the culture medium at a high temperature of 120° C for 20 min under high pressure; then, as chlorella, Chlorella regularis was added in such an amount of Vp = 0.3 mℓ/ℓ, and the chlorella was cultured in the same conditions as in Example 1.

As a result, after 40 hours of culturing, the cell concentration in the broth became Vp = 40 mℓ/ℓ and the chlorella was obtained in a high yield.

Having described our invention as related to the embodiment, it is our intention that the invention be not limited by any of the details of the description, unless otherwise specified, but rather be construed broadly within its spirit and scope as set out in the accompanying claims.

**Claims**

1. A method for culturing a chlorella aerobically in a culture medium containing an organic carbon source, which comprises using whey treated with a lactose-decomposing enzyme as the said organic carbon source.

2.  The method for culturing a chlorella as claimed in claim 1, wherein whey is pretreated with a lactose-decomposing enzyme to be used as a culture medium component before a chlorella is cultured.

3.  The method for culturing a chlorella as claimed in claim 1, wherein the treatment of whey with a lactose-decomposing enzyme and the culturing of a chlorella are allowed to proceed simultaneously.

4.  The method for culturing a chlorella as claimed in claim 1, wherein the lactose-decomposing enzyme is a $\beta$-galactosidase.

5.  The method for culturing a chlorella as claimed in claim 1, wherein the lactose-decomposing enzyme is a galactosyltransferase.

6.  The method for culturing a chlorella as claimed in claim 1, wherein an auxiliary nutrient, a nitrogen source, or a vitamin source is added into the culture medium.

7.  The method for culturing a chlorella as claimed in claim 1, wherein an iron salt is added into the culture medium.

8.  The method for culturing a chlorella as claimed in claim 1, wherein a metal salt is added into the culture medium.

9.  The method for culturing a chlorella as claimed in claim 1, wherein urea, a nitrate, or an ammonium salt is added into the culture medium.

10.  The method for culturing a chlorella as claimed in claim 1, wherein the chlorella is selected from the group consisting of Chlorella vulgaris, Chlorella protothecoides, Chlorella regularis, and Chlorella pyrenoidosa.

11.  The method for culturing a chlorella as claimed in claim 1, wherein the culture medium for culturing a chlorella is kept at pH of 6 to 8 and at temperature of 25 to 37° C.

12.  The method for culturing a chlorella as claimed in claim 1, wherein air is passed through the culture medium in an amount of 0.5 to 1 VVM.

# F I G . 1

Vp (mℓ/ℓ) vs Cultured Time (hr.)

o This Invention
× Comparative Example

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 89, no. 11, September 11, 1978, Columbus, Ohio, USA H. ENDO et al. "Utilization of whey" page 308, left column, abstract-No. 88 831t & JP-A-78-50 385 | 1,6-8, 10,11 | C 12 N 1/12 //(C 12 N 1/12 C 12 R 1:89) |
| X | CHEMICAL ABSTRACTS, vol. 91, no. 19, November 5, 1979, Columbus, Ohio, USA O.N. AL'BITSKAYA et al. "Culture medium for culti- vating microalgae" page 333, right column, abstract-No. 154 220n & SU-A-678 065 | 1,6,8, 9 | |

TECHNICAL FIELDS
SEARCHED (Int. Cl.5)

C 12 N

The present search report has been drawn up for all claims

| Place of search | Date of completion of the search | Examiner |
|---|---|---|
| VIENNA | 22-05-1991 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)